# EUROPÄISCHE PATENTSCHRIFT

(11) **EP 1 563 265 B1**
(45) Veröffentlichungstag und Bekanntmachung des Hinweises auf die Patenterteilung: **25.04.2007**
(21) Anmeldenummer: 03789025.8
(22) Anmeldetag: 11.11.2003
(51) Int. Cl.: G01G 19/14, G01G 17/04, A61B 5/20, A61M 1/00

(54) **SEKRETSAMMELVORRICHTUNG**
SECRETION-COLLECTING APPARATUS
DISPOSITIF DE RECUPERATION DE SECRETIONS

(30) Priorität: 19.11.2002 DE 20217847 U
(43) Veröffentlichungstag der Anmeldung: 17.08.2005
(73) Patentinhaber: B. BRAUN MELSUNGEN AG, 34212 Melsungen (DE)
(72) Erfinder: HAACKE, Claus, 34212 Melsungen (DE); PAETZOLD, Matthias, 34212 Melsungen (DE)
(74) Vertreter: Selting, Günther
(86) Internationale Anmeldenummer: PCT/EP2003/012568
(87) Internationale Veröffentlichungsnummer: WO 2004/046666

(56) Entgegenhaltungen:
- DE-A- 3 544 031
- US-A- 4 712 567
- US-A- 5 756 940
- PATENT ABSTRACTS OF JAPAN vol. 006, no. 018 (P-100), 2. Februar 1982 (1982-02-02) & JP 56 140217 A (NIPPON DENKI SANEI KK), 2. November 1981 (1981-11-02)

## Beschreibung

Die Erfindung betrifft eine Sekretsammelvorrichtung mit einem Gehäuse, das eine Anzeigevorrichtung aufweist und eine Wägevorrichtung trägt, wobei die Wägevorrichtung eine Aufhängevorrichtung für einen mit einem Überleitschlauch versehenen Auffangbehälter aufweist.

Eine Sekretsammelvorrichtung mit Wägevorrichtung dient zur Mengenerfassung von Körpersekret, wie beispielsweise Urin, Wundsekret, Pleurasekret, Aszitesflüssigkeit, Magensaft usw. Sekrete dieser Art, welche den Körper des Patienten verlassen, müssen zur Erstellung einer Fluidbilanz (Einfuhr-Ausfuhr-Bilanz) mit den durch Infusion in den Körper geleiteten Flüssigkeiten verrechnet werden, um den Wasser- oder Elektrolyt-Haushalt des Patienten steuern zu können.

In US-PS 4,448,207 ist eine Sekretsammelvorrichtung beschrieben, bei der der Füllstand des Auffangbehälters mittels Ultraschallmessung ermittelt wird, wobei aufgrund des gemessenen Füllstandes und der gespeicherten Querschnittsabmessungen des Behälters das Flüssigkeitsvolumen durch einen Computer berechnet wird.

US-PS 4,051,431 beschreibt eine Füllstandsmessung an einem Auffangbehälter durch kapazitive Pegelbestimmung, wobei ein starrer Behälter verwendet wird, dessen Volumen bekannt ist.

US-PS 4,314,484 beschreibt eine Vorrichtung, bei welcher die gesammelte Flüssigkeit in einen Behälter tropft und die Anzahl der Tropfen gezählt wird, um anhand der bekannten Tropfengröße das gesamte Volumen zu ermitteln.

Die genaueste Bestimmung der Flüssigkeitsmenge erfolgt generell durch eine Gewichtsmessung. Da hierbei jedoch die Genauigkeit des ermittelten Volumens von der Genauigkeit der Gewichtsmessung abhängt, ist es wichtig, dass nur das Gewicht der Flüssigkeit gemessen wird und dass keine Verfälschung durch das Gewicht des Auffangbehälters und durch Zug- oder Schiebekräfte erfolgt, die von dem Überleitschlauch auf den Auffangbehälter übertragen werden können. Die Gewichtsmessung hat den Vorteil, dass im Gegensatz zur direkten Volumenmessung keine besonderen Behälter erforderlich sind.

In DE 35 44 031 A1 ist eine Vorrichtung beschrieben, bei der an einem Gehäuse eine Wägevorrichtung vorgesehen ist, an die der Auffangbehälter angehängt werden kann. Das Ende des Überleitschlauchs wird an dem Gehäuse festgehalten und mit dem Auffangbehälter verbunden.

Der Oberbegriff des Patentanspruchs 1 geht aus von einer Sekretsammelvorrichtung wie sie in JP-A-56140217 beschrieben ist. Diese Vorrichtung weist eine in einem Gehäuse angebrachte Wägevorrichtung auf, an der ein Auffangbehälter aufgehängt werden kann. Seitlich an dem Gehäuse ist eine Fixiervorrichtung angebracht, die den zu dem Auffangbehälter führenden Überleitschlauch festhält und in definierter Ausrichtung in einem Zwischenabschnitt positioniert.

US-A-4,712,567 beschreibt eine Sekretsammelvorrichtung. An der Unterseite eines Gehäuses ist die Aufhängevorrichtung einer Wägevorrichtung angeordnet. An der Aufhängevorrichtung wird ein Sekretbeutel aufgehängt. Ein Überleitschlauch des Sekretsammelbehälters ist mit einer Hülse versehen, die mit einer Klemmvorrichtung an der Unterseite des Gehäuses festgeklemmt wird. Der Abschnitt des Überleitschlauchs zwischen Aufhängevorrichtung und Fixiervorrichtung wird thermogeformt oder derart getempert, dass er in derselben Vertikalebene liegt wie der Sekretsammelbeutel. Das Schlauchstück zwischen Aufhängevorrichtung und Fixiervorrichtung ist relativ kurz und ist in einem Bereich geringer Höhe geführt, so dass die Möglichkeit der Behinderung der Wägevorrichtung durch den Überleitschlauch besteht, wodurch die Genauigkeit des Messergebnisses beeinträchtigt wird.

Der Erfindung liegt die Aufgabe zugrunde, eine einfache und kostengünstige Sekretsammelvorrichtung mit Gewichtsmessung zu schaffen, bei welcher eine Übertragung von Zug- und Schiebekräften auf die Wägvorrichtung mit einfachen Mitteln verhindert wird.

Die Sekretsammelvorrichtung der vorliegenden Erfindung wird durch den Patentanspruch 1 definiert. Hiernach ist die Fixiervorrichtung oberhalb des Gehäuses an einem über das Gehäuse hinausragenden Halter derart angebracht, dass der Überleitschlauch in einem Bogen von der Fixiervorrichtung zu dem an der Aufhängevorrichtung befestigten Auffangbehälter führt.

Durch die Fixiervorrichtung wird ein Zwischenabschnitt des Überleitschlauchs fixiert, wobei sich eine ausreichend große Schlauchlänge zwischen dem fixierten Zwischenabschnitt und dem Schlauchende befindet. Diese Schlauchlänge ermöglicht infolge ihrer Flexibilität eine freie Anpassung an die jeweilige Position des Auffangbehälters, ohne die Wägevorrichtung wesentlich zu belasten. Die Fixiervorrichtung bewirkt eine Zug- und Spannungsentlastung der anschließenden Schlauchlänge, die somit im Wesentlichen unbelastet von Zug- oder Schiebekräften bleibt. Die im Bereich dieser Schlauchlänge eventuell auftretenden Verformungskräfte sind gleichbleibend, so dass sie das Messergebnis, das auf einer Gewichtsdifferenz beruht, nicht beeinflussen.

Der Halter ist oberhalb der Aufhängevorrichtung für den Auffangbehälter und mit Abstand von dieser angeordnet. Der Überleitschlauch wird dabei mit einem relativ langen linearen vertikalen Abschnitt von oben her an die Aufhängevorrichtung herangeführt, wobei die Schlauchführung oberhalb des geraden Abschnitts in einem Bogen erfolgen kann.

Der Auffangbehälter kann ein elastischer Beutel sein, der Aufhängeelemente aufweist, die mit der Aufhängevorrichtung in Eingriff kommen, wodurch der Auffangbehälter an der Wägevorrichtung bewegbar aufgehängt ist.

Gemäß einer bevorzugten Ausführungsform der Erfindung ist an dem Überleitschlauch ein Kupplungsteil angebracht, das mit einem Kupplungsteil der Fixiervorrichtung zusammengreift. Das Kupplungsteil des Schlauches befindet sich in einem Abstand von dem Auffangbehälter, der gleich dem 3- bis 15-fachen, vorzugsweise gleich dem 10-fachen des Außendurchmessers des Überleitschlauchs ist. Die Kupplungsteile fixieren den betreffenden Schlauchabschnitt in allen Raumrichtungen. Auf diese Weise kann der Schlauch in einem Bogen geführt werden, ohne die Bewegung des Auffangbehälters bei Veränderung seines Gewichts zu beeinflussen. Die Festlegung von Abstand und Raumrichtung des schlauchseitigen Kupplungsteils werden beeinflusst von den Biegeeigenschaften des Schlauchs. Diese setzen sich zusammen aus dem materialtypischen Elastizitätsmodul, der Wandstärke und dem Schlauchdurchmesser. Es kann zweckmäßig sein, an unterschiedlichen Schlauchtypen Kupplungsteile mit unterschiedlicher Schlauchorientierung vorzusehen.

Für verschiedene Methoden der Sekretdrainage werden unterschiedliche Schlauchtypen verwendet, wobei sowohl Härte als auch Lumen und Wandstärke voneinander abweichen können. Das Anbringen von Schlauchtypen, die nicht an die Vorrichtung angepasst sind, mit undefiniertem Abstand und Raumrichtung des Kupplungsteils muss vermieden werden. Daher sind die Kupplungsteile gewissermaßen als Schlüssel und Schlüsselloch ausgebildet, so dass nur ein bestimmtes schlauchseitiges Kupplungsteil mit einem bestimmten Kupplungsteil der Fixiervorrichtung züsammenpasst.

Zweckmäßigerweise trägt das eine Kupplungsteil eine Kodierung, auf die ein Sensor des anderen Kupplungsteils anspricht. Auf diese Weise können durch die Kodierung Informationen über das Volumen des Auffangbehälters, den Überleitschlauch und andere Parameter an eine in dem Gehäuse enthaltene Steuer- und Überwachungseinrichtung übertragen werden.

Weiterhin muss sichergestellt werden, dass die Kupplungsteile sich nicht verdrehen können und in der erforderlichen Tiefe fest ineinander gesteckt sind, so dass sie nicht durch versehentliche Bewegungen des Schlauches herausgezogen werden können. Die Messung darf erst dann gestartet werden, wenn die Kupplungsteile fest ineinander gesteckt sind und zusammengreifen. Bei laufender Messung, wenn beispielsweise der Auffangbehälter aus der Messapparatur entfernt werden muss, soll auch dieser Situationswechsel für die Elektronik erkennbar sein. Ebenso wird der Elektronik zu Kontrollzwecken erkennbar gemacht, welcher Typ von Auffangbehälter eingehängt wurde und wie groß dessen maximales Füllvolumen ist.

Durch seitliche Zähne oder Höcker auf dem eingesteckten Kupplungsteil, welche mit Aussparungen, im anderen Kupplungsteil korrespondieren, wird eine definierte Lage des fixierten Schlauchabschnitts gewährleistet. Zur Erkennung des korrekten Ineinandergreifens der Kupplungsteile kann an dem einen Kupplungsteil eine Kodierung und an dem anderen Kupplungsteil ein die Kodierung erkennender Detektor vorgesehen sein. Das System aus Kodierung und Detektor kann nach unterschiedlichen Prinzipien funktionieren, beispielsweise mit elektrischen Kontaktschaltern, Lichtschranken, Farbdetektierung, Transponderkodierungen oder Erkennung über elektromagnetische Eigenschaften (Impedanz oder Kapazität). Das System erlaubt es, den jeweiligen Auffangbehälter bzw. Überleitschlauch zu erkennen und einer einprogrammierten Liste zuzuordnen.

Im Folgenden werden unter Bezugnahme auf die Zeichnungen Ausführungsbeispiele der Erfindung näher erläutert, jedoch ohne hierdurch den Schutzbereich zu beschränken, der durch die Patentansprüche bestimmt wird.

Es zeigen:

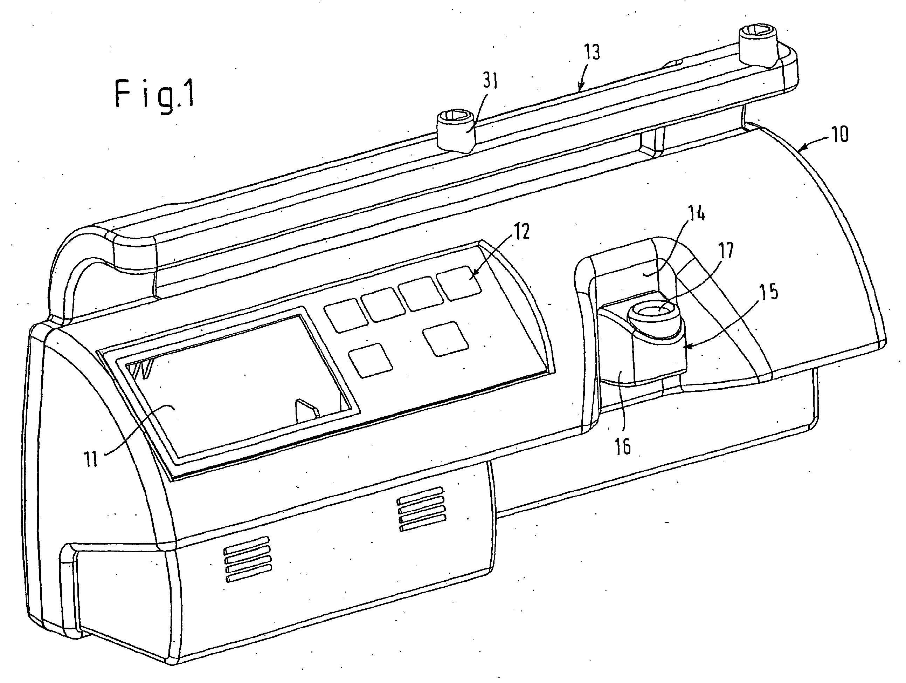

- Fig. 1: eine perspektivische Darstellung der Sekretsammelvorrichtung,
- Fig. 2: eine Stirnansicht der Sekretsammelvorrichtung,
- Fig. 3: einen Querschnitt durch das Gehäuse und einen Teil der Wägevorrichtung, wobei ein Auffangbehälter mit Überleitschlauch eingesetzt ist,
- Fig. 4: eine Draufsicht auf ein Gehäuse mit einer anderen Ausführungsform der Aufhängevorrichtung in Kombination mit einer alternativen Ausführung des Auffangbehälters,
- Fig. 5: eine weitere Ausführungsform des Auffangbehälters mit einer alternativen Aufhängung,
- Fig. 6: ein Ausführungsbeispiel der Kupplung zwischen Fixiervorrichtung und Überleitschlauch,
- Fig. 7: ein weiteres Ausführungsbeispiel der Kupplung mit Kodierung und Detektor, und
- Fig. 8: ein weiteres Ausführungsbeispiel der Kupplung mit Kodierung und Detektor.

Die als Ausführungsbeispiel dargestellte Sekretsammelvorrichtung weist ein Gehäuse 10 auf, das mit einer Anzeigevorrichtung 11 und einem Bedienfeld 12 versehen ist. Das Gehäuse weist ferner einen Tragegriff 13 auf, der in Längsrichtung des Gehäuses verläuft. Der Tragegriff ermöglicht sowohl das Tragen des Gehäuses als auch dessen Aufhängung.

In einer Ausnehmung 14, die einen zur Vorderseite des Gehäuses hin offenen Schacht bildet, befindet sich eine Aufhängevorrichtung 15, die aus einem vertikal bewegbaren Block 16 mit einer vertikalen Einsteckhülse 17 besteht. An der Aufhängevorrichtung 15 wird der Auffangbehälter 20 befestigt. Dieser weist bei dem vorliegenden Ausführungsbeispiel einen flexiblen Sekretsammelbeutel 21 auf, der an seinem oberen Ende mit einem Überleitschlauch 22 verbunden ist. An dem oberen Ende des Sekretsammelbeutels 21 ist ein Halter 23 befestigt, der einen in die Einsteckhülse 17 passenden Zapfen 24 aufweist. Dadurch kann der Auffangbehälter 20 an der Aufhängevorrichtung 15 in definierter Position angebracht werden, wobei der Halter 23 das Gewicht des Auffangbehälters auf die Aufhängevorrichtung 15 überträgt.

Die Aufhängevorrichtung 15 ist Bestandteil einer Wägevorrichtung 25. Sie ist an einer Wägezunge 26 angebracht, die in das Innere des Gehäuses 10 hineinragt und dort an einem Wägebalken 27 befestigt ist. Die Wägezunge 26 ragt durch eine Öffnung 28 der Gehäusewand derart hindurch, dass sie vertikale Bewegungen ausführen kann.

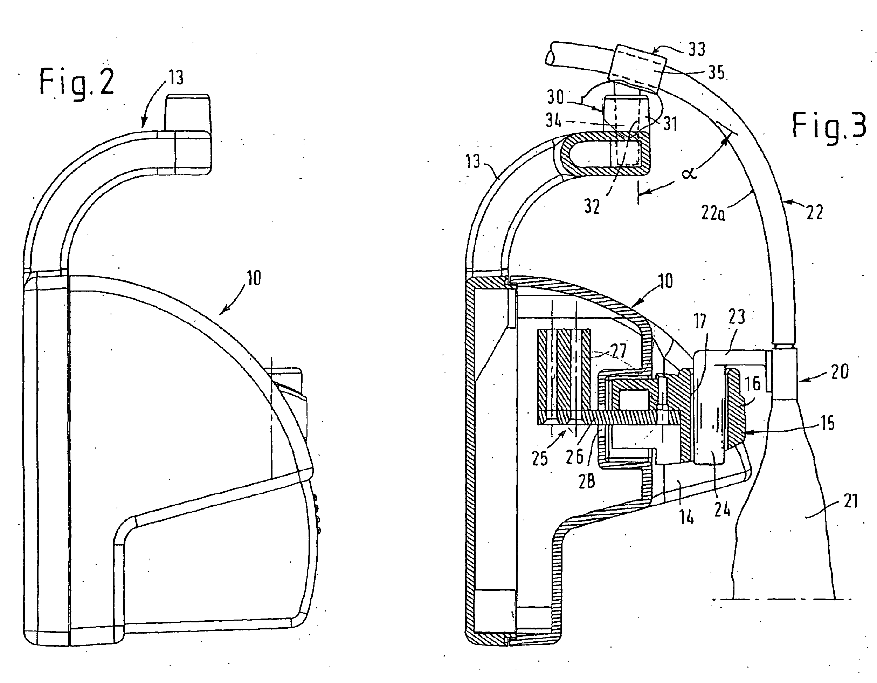

An dem Tragegriff 13, der Bestandteil des Gehäuses 10 ist, ist eine Fixiervorrichtung 30 für den Überleitschlauch 22 vorgesehen. Die Fixiervorrichtung 30 besteht aus einem Kupplungsteil 31 mit einer vertikalen Einsteckhülse 32. Das Kupplungsteil 31 greift mit einem Kupplungsteil 33 zusammen, das an dem Überleitschlauch 22 fest angebracht ist und einen in die Einsteckhülse 32 passenden Zapfen 34 aufweist. Der Zapfen 34 steht von einer Führungshülse 35 ab, die einen kurzen Zwischenabschnitt des Überleitschlauchs 22 umgibt und dessen Richtung bzw. Orientierung bestimmt. Die Hülse 35 bildet mit dem Zapfen 34 einen festen spitzen Winkel α, wodurch die zwischen dem Kupplungsteil 33 und dem Halter 23 liegende Schlauchlänge 22a des Überleitschlauchs 22 zunächst unter einem Winkel von annähernd 45° schräg nach unten gerichtet wird und anschließend linear vertikal zu dem Halter 23 übergeht. Diese Schlauchlänge erhält somit, seitlich aus einer Stirnansicht des Gehäuses betrachtet, einen bogenförmigen Verlauf. Die Fixiervorrichtung 30 ist bezogen auf die Aufhängevorrichtung 15 zurückliegend angeordnet. Der in Figur 3 erkennbare Bogen des Überleitschlauchs 22 dient als Reserve oder Pufferelement zum Ausgleich der Vertikalbewegungen der Aufhängevorrichtung 15. Dabei werden vertikale Kräfte auf die Aufhängevorrichtung weitgehend vermieden.

Die Kupplungsteile 31 und 33 greifen verdrehungssicher zusammen und zwar derart, dass der Zapfen 34 in die Einsteckhülse 32 nur in einer vorher bestimmten Orientierung hineinpasst. Dies wird dadurch erreicht, dass Zapfen und Einsteckhülse komplementäre Querschnittsgestalt haben, die beispielsweise rechteckig, quadratisch, dreieckförmig oder kreuzförmig sein kann. Eine eindeutig definierte Orientierung wird beispielsweise durch ein Profil in der Form eines gleichschenkligen Dreiecks erzeugt. Durch die definierte und verdrehungssichere Orientierung des Kupplungsteils 33 ist sichergestellt, dass die Schlauchlänge 22a einen definierten Bogen bildet.

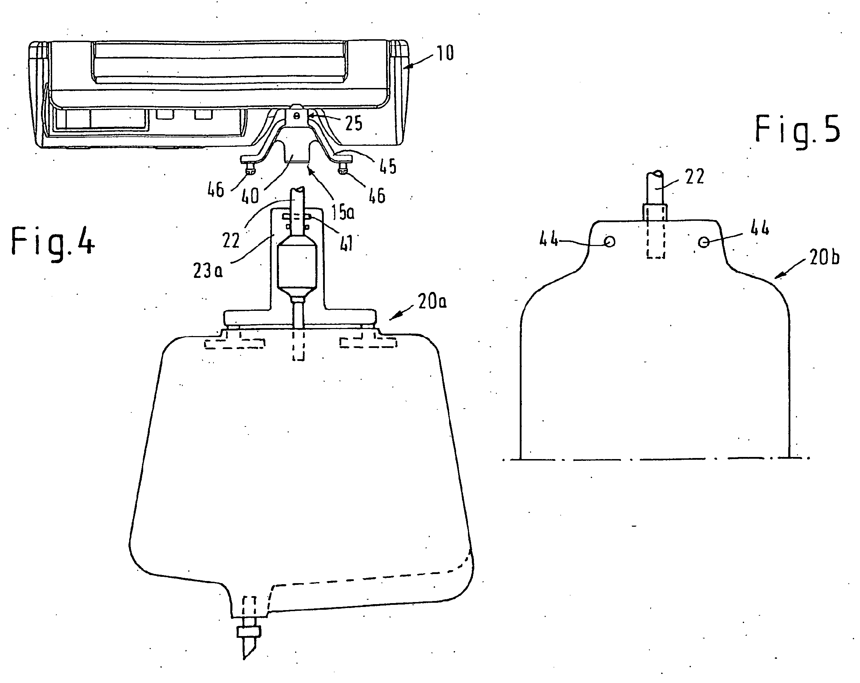

Figur 4 zeigt eine alternative Ausführungsform der Aufhängevorrichtung 15a, die an der Wägevorrichtung 25 befestigt ist. Die Aufhängevorrichtung 15a weist in diesem Fall eine vorstehende horizontale Zunge 40 auf. Der Halter 23a des Auffangbehälters 20a hat einen horizontalen Schlitz 41, der auf die Zunge 40 aufgeschoben wird, wodurch der Auffangbehälter 20a an der Aufhängevorrichtung lösbar angebracht wird.

Die Aufhängevorrichtung 15a kann ferner dazu benutzt werden, den in Figur 5 dargestellten Typ von Auffangbehälter 20b anzubringen. Dieser weist zwei Löcher 44 auf. Die Aufhängevorrichtung 15a ist mit einem Bügel 45 versehen, von dem zwei Zapfen 46 mit verdickten Köpfen nach vorne abstehen. Die Löcher 44 können auf die Zapfen 46 aufgeschoben werden, um den Auffangbehälter 20b, bei dem es sich um einen flexiblen Beutel handelt, an der Aufhängevorrichtung zu befestigen. Es sind auch andere Formen von Aufhängevorrichtung und Auffangbehälter möglich. Wichtig ist, dass die Aufhängevorrichtung das gesamte Gewicht des Auffangbehälters trägt und diesen in definierter Ausrichtung hält, so dass der Überleitschlauch 22, der einerseits durch die Fixiervorrichtung 30 und andererseits durch die Aufhängevorrichtung 15 festgelegt ist, einen definierten Verlauf erhält.

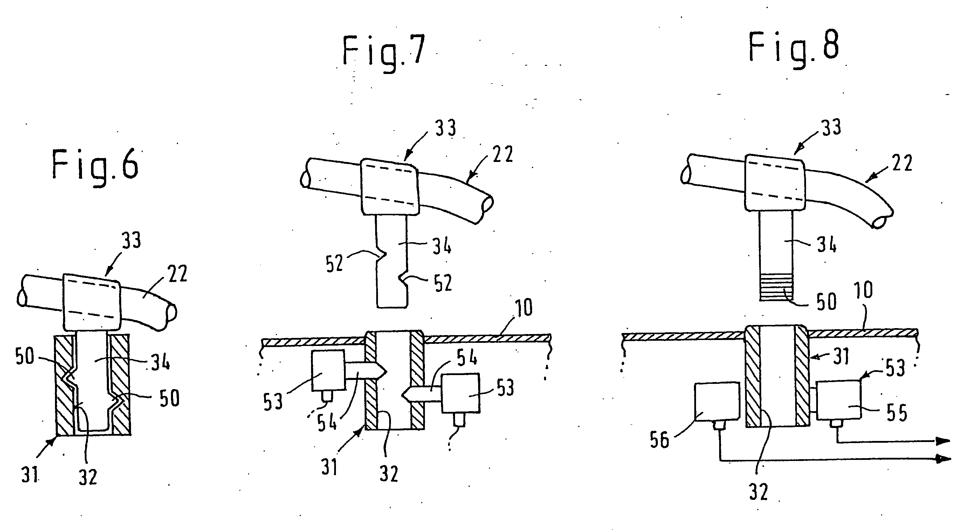

Figur 6 zeigt ein Ausführungsbeispiel, bei dem der Zapfen 34 des Kupplungsteils 33 Vorsprünge 50 hat, die in entsprechenden Ausnehmungen der Einsteckhülse 32 einrasten, so dass ein ordnungsgemäßes Zusammengreifen der Kupplungsteile 31 und 33 nur vorliegt, wenn der Zapfen 34 in voller Tiefe eingesteckt ist. Außerdem ist eine (nicht dargestellte) Verdrehsicherung vorgesehen, die bewirkt, dass der Zapfen nur in der richtigen Drehposition eingesteckt werden kann.

Bei dem Ausführungsbeispiel von Figur 7 ist der Zapfen 34 mit einer Kodierung 52 versehen, die hier die Form von Kerben hat. An der Einsteckhülse 32 sind Detektoren 53 in Form von Mikroschaltern vorgesehen, deren Tastelemente 54 auf die Kodierungen 52 ansprechen. Die Tastelemente 54 sind federnd vorgespannte Schaltstifte, die von der Außenfläche des Zapfens 34 zurückgedrängt werden und mit ihren Spitzen in die Kerben eindringen und somit die korrekte Position einer Kerbe erkennen. Wenn sämtliche Detektoren 53 die jeweilige Kodierung 52 erkannt haben, wird der Steuereinrichtung signalisiert, dass die beiden Kupplungsteile 31 und 33 in korrekter Weise zusammengesteckt wurden. Daraufhin beginnt der Mengenmessvorgang.

Bei dem Ausführungsbeispiel von Figur 8 ist ebenfalls an dem Zapfen 34 eine Kodierung 50 und an der Einsteckhülse 32 ein entsprechender Detektor 53 vorgesehen, der auf die Kodierung 50 anspricht, wenn diese sich in der korrekten Position befindet. Der Detektor 53 besteht bei diesem Ausführungsbeispiel aus einer Lichtquelle 55 und einem Lichtempfänger 56. Die Wand der Einsteckhülse 32 ist lichtdurchlässig und befindet sich im Lichtweg zwischen Lichtquelle 55 und Lichtempfänger 56. Die Kodierung 50 ist ein lichtdurchlässiges Teil.

Anstelle der beschriebenen Kombination von Kodierung und Detektor können auch andere Kombinationen verwendet.werden, wie sie in der nachfolgenden Tabelle aufgeführt sind, die jedoch nicht vollständig ist:

**Tabelle**

| *Kodierung* | *Detektor* |
|---|---|
| 1. Gefärbtes, durchsichtiges Polymerteil | Lichtschranke |
| 2. Ferritkern | Elektrische Spule, Induktivität |
| 3. Dielektrikum | Plattenkondensator |
| 4. Microchip | Lesegerät |
| 5. Transponder | Lesegerät |
| 6. Barcode | Barcodelesegerät |
| 7. Magnetplättchen | Magnetsensor |

Die Erfindung ist insbesondere vorteilhaft für Sekretableitungs- und Bilanziervorrichtungen, welche für verschiedene Typen von Auffangbehältern und/oder für verschiedene Sekretarten mit verschiedenen Schlaucharten verwendbar sind. Beispielsweise können sich die Schläuche durch Unterschiede im Material, der Elastizität oder in den Abmessungen von Wandstärke und Durchmesser unterscheiden. Die Schläuche werden entsprechend ihren mechanischen Eigenschaften an speziellen Punkten, deren Abstände von der jeweiligen Aufhängevorrichtung am Wägebalken festgelegt werden, auf der Geräteaußenseite suspendiert. Diese Festhaltepunkte sind produktspezifisch. Zur Sicherung, dass nur passende Sammelbehältnisse Verwendung finden und zur Kontrolle, ob der Schlauch ordnungsgemäß fixiert wurde, wird die Befestigung an der Fixiervorrichtung automatisch überwacht.

Durch die Erfindung wird sichergestellt, dass nur zugelassene Kombinationen aus Auffangbehälter und Überleitschlauch verwendet werden. Die Elektronik erkennt nicht nur das Vorhandensein eines Auffangbehälters, sondern auch dessen Typ. Für jeden Behältertyp sind bestimmte Charakteristiken wie Eichkurve, maximales Volumen und andere, passend zu der jeweiligen Software, abgespeichert. Der Start der Messung erfolgt nur nach anwendungsgerechter Suspension des Schlauchs.

## Patentansprüche

1. Sekretsammelvorrichtung mit einem Gehäuse' (10), das eine Wägevorrichtung (25) enthält, wobei die Wägevorrichtung eine Aufhängevorrichtung (15) für einen mit einem Überleitschlauch (22) verbundenen Auffangbehälter (20), und einer an dem Gehäuse (10) entfernt von der Aufhängevorrichtung (15) angeordneten Fixiervorrichtung (30) zur definierten Positionierung und Ausrichtung eines Zwischenabschnitts des Überleitschlauchs (22),
**dadurch gekennzeichnet**
**dass** die Fixiervorrichtung (30) oberhalb des Gehäuses (10) an einem über das Gehäuse hinausragenden Halter (13) derart angebracht ist, dass der Überleitschlauch (22) in einem Bogen von der Fixiervorrichtung (30) zu dem an der Aufhängevorrichtung (15) befestigten Auffangbehälter (20) führt.

2. Sekretsammelvorrichtung nach Anspruch 1, **dadurch gekennzeichnet, dass** die Fixiervorrichtung (30) in Bezug auf die an einer Frontseite des Gehäuses (10) angeordnete Aufhängevorrichtung (15) zurückgesetzt und seitlich versetzt angeordnet ist.

3. Sekretsammelvorrichtung nach Anspruch 1 oder 2, **dadurch gekennzeichnet, dass** an dem Überleitschlauch (22) ein Kupplungsteil (33) angebracht ist, das mit einem Kupplungsteil (31) der Fixiervorrichtung (30) in definierter unveränderlicher Ausrichtung lösbar zusammengreift.

4. Sekretsammelvorrichtung nach Anspruch 3, **dadurch gekennzeichnet, dass** die beiden Kupplungsteile (31,33) verdrehsicher zusammengreifen.

5. Sekretsammelvorrichtung nach Anspruch 3, **dadurch gekennzeichnet, dass** die beiden Kupplungsteile (31,33) rastend zusammengreifen.

6. Sekretsammelvorrichtung nach Anspruch 3, **dadurch gekennzeichnet, dass** das eine Kupplungsteil (33) ein Zapfen (34) und das andere Kupplungsteil (31) eine dazu passende Einsteckhülse (32) ist.

7. Sekretsammelvorrichtung nach Anspruch 3, **dadurch gekennzeichnet, dass** das eine Kupplungsteil (33) eine Kodierung (52) aufweist, auf die ein Detektor (53) des anderen Kupplungsteils (31) anspricht.

8. Sekretsammelvorrichtung nach einem der Ansprüche 1 - 7, **dadurch gekennzeichnet, dass**'der Auffangbehälter (20) eine Kodierung aufweist, an die ein Detektor der Aufhängevorrichtung (15) anspricht.

9. Sekretsammelvorrichtung nach Anspruch 7 oder 8, **dadurch gekennzeichnet, dass** die Kodierung aus einem digitalen Speicher und der Detektor aus einem den Speicher berührungslos abfragenden Abfragegerät besteht.

## Claims

1. A secretion collecting apparatus comprising a housing (10) including a weighing device (25), the weighing device comprising a suspension device (15) for a collecting container (20) connected to a transfer hose (22), and a fixation device (30) provided at the housing (10) remote from the suspension device (15), for a defined positioning and orientation of an intermediate section of the transfer hose (22),
**characterized in that**,
above the housing (10), the fixation device (30) is attached to a holder (13) projecting beyond the housing, such that the transfer hose (22) extends in a bend from the fixation device (30) to the collection container (20) attached to the suspension device (15).

2. The secretion collecting apparatus of claim 1, **characterized in that** the fixation device (30) is offset backward and laterally with respect to the suspension device (15) provided at a front side of the housing (10).

3. The secretion collecting apparatus of claim 1 or 2, **characterized in that**, at the transfer hose (22), a coupling member (33) is attached which mates releasably with a coupling member (31) of the fixation device (30) in a defined invariable orientation.

4. The secretion collecting apparatus of claim 3, **characterized in that** the two coupling members (31, 33) mate with each other unrotatably.

5. The secretion collecting apparatus of claim 3, **characterized in that** both coupling members (31, 33) mate in a resting manner.

6. The secretion collecting apparatus of claim 3, **characterized in that** one coupling member (33) is a pin (34) and the other coupling member (31) is a mating plug-in socket (32).

7. The secretion collecting apparatus of claim 3, **characterized in that** said one coupling member (33) bears a code (52) to which a detector (53) of said other coupling member (31) responds.

8. The secretion collecting apparatus of one of claims 1-7, **characterized in that** the collecting container (20) bears a code to which a detector of the suspension device (15) responds.

9. The secretion collecting apparatus of claim 7 or 8, **characterized in that** the code is a digital memory and the detector is an interrogating device contactlessly interrogating the memory.

## Revendications

1. Dispositif de récupération de secrétions comprenant un boîtier (10) muni d'un dispositif de pesage (25), dans lequel le dispositif de pesage présente un dispositif d'accrochage (15) pour un récipient de récupération (20) relié à un tuyau de transfert (22) ainsi qu'un dispositif de fixation (30) disposé sur le boîtier (10) à une distance du dispositif d'accrochage (15) pour le positionnement et l'orientation définis d'un segment intermédiaire du tuyau de transfert (22), **caractérisé en ce que** le dispositif de fixation (30) est installé au-dessus du boîtier (10) sur un support (13) faisant saillie du boîtier de sorte que le tuyau de transfert (22) s'étend en forme d'arc du dispositif de fixation (30) au récipient de récupération (20) fixé au dispositif d'accrochage (15).

2. Dispositif de récupération de secrétions selon la revendication 1, **caractérisé en ce que** le dispositif de fixation (30) est en retrait et latéralement décalé par rapport au dispositif d'accrochage (15) disposé sur une face frontale du boîtier (10).

3. Dispositif de récupération de secrétions selon la revendication 1 ou 2, **caractérisé en ce que** le tuyau de transfert (22) présente un élément de couplage (33) qui coopère de façon amovible selon une orientation définie invariable avec un élément de couplage (31) du dispositif de fixation (30).

4. Dispositif de récupération de secrétions selon la revendication 3, **caractérisé en ce que** les deux éléments de couplage (31, 33) coopèrent sans jeu de torsion.

5. Dispositif de récupération de secrétions selon la revendication 3, **caractérisé en ce que** les deux éléments de couplage (31, 33) coopèrent de façon bloquante.

6. Dispositif de récupération de secrétions selon la revendication 3, **caractérisé en ce qu'**un élément de couplage (33) est un tourillon (34) et l'autre élément de couplage (31) est une douille femelle (32) correspondante.

7. Dispositif de récupération de secrétions selon la revendication 3, **caractérisé en ce qu'**un élément de couplage (33) présente un codage (52) auquel réagit un détecteur (53) de l'autre élément de couplage (31).

8. Dispositif de récupération de secrétions selon l'une quelconque des revendications 1 à 7, **caractérisé en ce que** le récipient de récupération (20) présente un codage auquel réagit un détecteur du dispositif d'accrochage (15).

9. Dispositif de récupération de sécrétions selon la revendication 7 ou 8, **caractérisé en ce que** le codage est une mémoire numérique et le détecteur est un appareil de lecture interrogeant la mémoire sans contact.
